# EUROPEAN PATENT SPECIFICATION

(11) **EP 2 303 923 B1**
(45) Date of publication and mention of the grant of the patent: **24.06.2015**
(21) Application number: 08875708.3
(22) Date of filing: 16.07.2008
(51) Int. Cl.: C07K 16/08, G01N 33/569, A61K 39/42

(54) **HUMAN CYTOMEGALOVIRUS NEUTRALISING ANTIBODIES AND USE THEREOF**
DAS HUMANE CYTOMEGALOVIRUS NEUTRALISIERENDE ANTIKÖRPER UND ANWENDUNG DAVON
ANTICORPS NEUTRALISANT LES CYTOMÉGALOVIRUS HUMAINS ET LEUR UTILISATION

(43) Date of publication of application: 06.04.2011
(73) Proprietor: Institute for Research in Biomedicine, 6500 Bellinzona (CH)
(72) Inventor: LANZAVECCHIA, Antonio, CH-6500 Bellinzona (CH); MACAGNO, Annalisa, CH-6500 Bellinzona (CH)
(74) Representative: Tuxworth, Pamela M.
(86) International application number: PCT/IB2008/002683
(87) International publication number: WO 2010/007463

(56) References cited:
- WO-A-2007/146024
- WO-A-2008/084410
- WO-A2-2006/002177
- WO-A2-2006/137931
- WANG DAI ET AL: "Human cytomegalovirus virion protein complex required for epithelial and endothelial cell tropism" PROCEEDINGS OF THE NATIONAL ACADEMY OF SCIENCES OF USA, NATIONAL ACADEMY OF SCIENCE, WASHINGTON, DC.; US, vol. 102, no. 50, 1 December 2005 (2005-12-01), pages 18153-18158, XP002463457 ISSN: 0027-8424
- ADLER B ET AL: "Role of human cytomegalovirus UL131A in cell type-specific virus entry and release" JOURNAL OF GENERAL VIROLOGY, SOCIETY FOR GENERAL MICROBIOLOGY, SPENCERS WOOD, GB, vol. 87, no. 9, 1 September 2006 (2006-09-01), pages 2451-2460, XP002500659 ISSN: 0022-1317
- RYCKMAN BRENT J ET AL: "Characterization of the human cytomegalovirus gH/gL/UL128-131 complex that mediates entry into epithelial and endothelial cells." JOURNAL OF VIROLOGY JAN 2008, vol. 82, no. 1, January 2008 (2008-01), pages 60-70, XP002519851 ISSN: 1098-5514
- ANDREONI K A ET AL: "Human CMV-IGIV (CytoGam) neutralizes human cytomegalovirus (HCMV) infectivity and prevents intracellular signal transduction after HCMV exposure.", TRANSPLANT INFECTIOUS DISEASE : AN OFFICIAL JOURNAL OF THE TRANSPLANTATION SOCIETY 2001 LNKD- PUBMED:11926746, vol. 3 Suppl 2, 2001, pages 25-30, ISSN: 1398-2273
- Anita a. Hamilton ET AL: "A Humanized Antibody against Human Cytomegalovirus (CMV) gpUL75 (gH) for Prophylaxis or Treatment of CMV Infections", The Journal of Infectious Diseases, vol. 176, no. 1, 1 July 1997 (1997-07-01), pages 59-68, XP055040900, ISSN: 0022-1899, DOI: 10.1086/514040

## Description

### TECHNICAL FIELD

This invention relates to antibodies having high potency in neutralizing human cytomegalovirus (hCMV) and being specific for a combination of the hCMV proteins UL128, UL130 and UL131A. Described herein are immortalised B cells that produce such monoclonal antibodies.

Also described herein is the epitope determined by a combination of the hCMV proteins UL128, UL130 and UL131A that the antibodies bind to as well as the use of the antibodies and the epitope in screening methods, diagnosis, prophylaxis and therapy of disease.

### BACKGROUND ART

Human cytomegalovirus (hCMV) is a widely distributed pathogen that may cause severe pathology in immunosuppressed adults and upon infection of the fetus and has been implicated in chronic diseases such as atherosclerosis. hCMV infects multiple cell types including fibroblasts, endothelial, epithelial and hematopoietic cells [1]. *In vitro* propagated attenuated strains of hCMV, which are being developed as candidate vaccines, have lost the tropism for endothelial cells, while retaining the capacity to infect fibroblasts [2]. Two viral glycoprotein complexes are believed to control the cellular tropism of hCMV. A complex of glycoproteins such as gH, gL and gO appears to be required for infection of fibroblasts, while a complex of gH, gL and proteins encoded by the UL131-UL128 genes is implicated in infection of endothelial cells, epithelial cells and dendritic cells [2-8].

Hyperimmune globulins are already commercialised for the prophylaxis of hCMV disease associated with transplantation and recent evidence indicates that they have therapeutic effect in pregnant women [9]. This therapeutic approach is limited by the low amount of neutralising antibody that can be transferred and for this reason the availability of human antibodies (such as human monoclonal antibodies) with high neutralising capacity would be highly desirable.

However the target of hCMV neutralising antibodies remains to be established. Although some antibodies to gH, gB and UL128 and UL130 gene products have demonstrated in vitro neutralizing activities [7, 10, 11] and an antibody to gH has been evaluated in clinical trials which were discontinued due to lack of therapeutic effects, the neutralizing potency of the monoclonal antibodies isolated so far is modest, since neutralization was observed at antibody concentrations ranging from 0.5 to 20 microgram/ml. Further, the current methods typically measure the neutralising potency of anti-hCMV antibodies using fibroblasts as target cells.

However, hCMV is also known to cause pathology by infecting other cell types such as endothelial, epithelial cells and leukocytes. Known antibodies to UL128 and UL130 show very low potency in neutralising infection of endothelial cells [7] and there do not appear to be any monoclonal antibodies available that would be capable of neutralising infection of non-fibroblast target cells with high potency. Antibodies against a CMV protein complex comprising pUL128 or pUL130 have been described [43], as have antibodies that neutralise hCMV, including antibodies specific for a combination of UL130 and UL131A [44].

There is therefore a need for antibodies that neutralise hCMV infection of non-fibroblast target cells with a higher potency, as well as the elucidation of the target(s) to which such antibodies bind.

### SUMMARY OF INVENTION

The invention is based, in part, on the discovery of novel antibodies that neutralise hCMV with high potency as well as novel epitopes to which the antibodies of the invention bind. Accordingly, in one embodiment, the invention comprises an isolated antibody, or an antigen binding fragment thereof, that is specific for a combination of hCMV proteins UL128, UL130 and UL131A, the combination forming the epitope recognized by said antibody or binding fragment, and that neutralizes infection of endothelial cells, retinal cells, or dendritic cells by human cytomegalovirus (hCMV), wherein the antibody or fragment comprises CDR1, CDR2, and CDR3 heavy chain sequences as set forth in SEQ ID NO: 1, SEQ ID NO: 2, and SEQ ID NO: 3 respectively, and CDR1, CDR2, and CDR3 light chain sequences as set forth in SEQ ID NO: 4, SEQ ID NO: 5, and SEQ ID NO: 6 respectively.

In another embodiment the invention comprises a nucleic acid molecule encoding an antibody or an antibody fragment of the invention.

In yet another embodiment the invention comprises a vector comprising a nucleic acid molecule encoding an antibody or an antibody fragment of the invention.

In a further embodiment the invention comprises a cell comprising a vector comprising a nucleic acid molecule of the invention.

Also described herein is an immortalised B cell clone expressing an antibody having high potency in neutralising hCMV, wherein said antibody is specific for a combination of the hCMV proteins UL128, UL130 and UL131A.

Also described herein are an epitope which binds to an antibody of the invention, an immunogenic polypeptide comprising an epitope which binds to an antibody of the invention, and a ligand which binds to an epitope which binds to an antibody of the invention.

Described herein are methods for producing antibodies having high potency in neutralising hCMV, wherein said antibody is specific for a combination of the hCMV proteins UL128, UL130 and UL131A. The method comprises (i) culturing an immortalised B cell clone expressing an antibody of the invention and (ii) isolating antibodies from the B cell.

In another embodiment the invention comprises a pharmaceutical composition comprising an antibody or an antibody fragment of the invention, and a pharmaceutically acceptable diluent or carrier. Also described are pharmaceutical compositions comprising a nucleic acid of the invention, an immortalised B cell clone expressing an antibody of the invention, or an immunogenic polypeptide comprising an epitope which binds to an antibody or an antibody fragment of the invention.

In a further embodiment the invention comprises the use of an antibody or fragment, nucleic acid, or cell or composition of the invention in the manufacture of a medicament for the prevention or treatment of hCMV infection; and an antibody or fragment or the composition of the invention for use in the prevention or treatment of hCMV infection.

Described herein is a kit for the diagnosis of hCMV infection comprising antibodies or antibody fragments of the invention, a nucleic acid encoding an antibody or an antibody fragment of the invention, or an epitope which binds to an antibody of the invention.

Also described herein is a method for preparing a recombinant cell. The method comprises: (i) sequencing nucleic acid from an immortalised B cell clone expressing an antibody of the invention and (ii) using the sequence information obtained from step (i) to prepare nucleic acid for inserting into an expression host in order to permit expression of the antibody of interest in that host.

Also described is a method for producing antibodies having high potency in neutralising hCMV and specific for a combination of the hCMV proteins UL 128, UL1 30 and UL131A. The method comprises culturing or sub-culturing an expression host obtainable by the method described above and, optionally, purifying the antibody of interest.

Also described is a method of screening for polypeptides that can induce an immune response against hCMV, comprising screening polypeptide libraries using an antibody or an antibody fragment of the invention.

### BRIEF DESCRIPTION OF DRAWINGS

Figure 1 shows a FACS analysis which demonstrates that the human monoclonal antibody (mAb) 6G4 recognises an epitope determined by a combination of the hCMV proteins UL128, UL130 and UL131A.

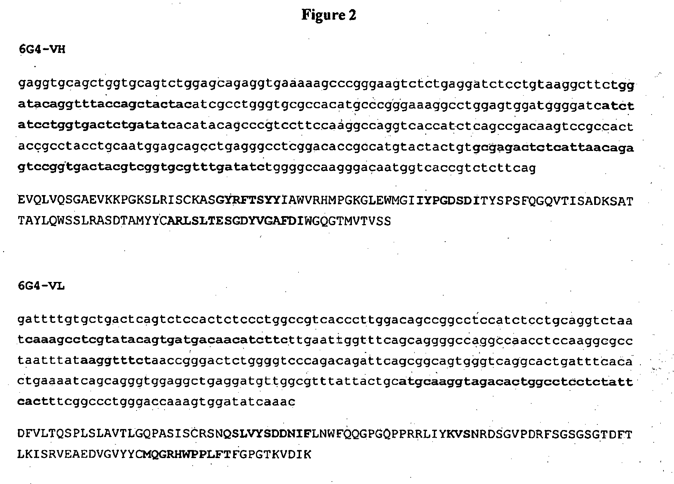

Figure 2 shows the nucleotide and amino acid sequences of the heavy and light chains of the antibody 6G4. The CDR sequences are in bold.

### DETAILED DESCRIPTION OF THE INVENTION

The invention is based on the production of antibodies and antibody fragments that neutralise hCMV infection and which have a particularly high potency in neutralising hCMV infection. Such antibodies are desirable, as only low concentrations are required in order to neutralise a given amount of virus. This facilitates higher levels of protection whilst administering lower amounts of antibody. Human monoclonal antibodies are also included within the scope of the invention. The inventors have discovered that antibodies directed to a combination of the hCMV proteins UL128, UL130 and UL131A are particularly effective in neutralising hCMV. Without being bound to any theory, this combination may be a precise complex of UL128, UL130 and UL131A forming a unique epitope recognised by the antibody.

Also described herein is the characterisation of the epitope to which the antibodies bind and the use of that epitope in raising an immune response.

Also described herein is various methods and uses involving the antibodies of the invention and the epitopes to which they bind.

### Antibodies of the invention

The invention relates to monoclonal or recombinant antibodies having particularly high potency in neutralising hCMV. The invention also relates to fragments of these monoclonal or recombinant antibodies, particularly fragments that retain the antigen-binding activity of the antibodies. In this specification, by "high potency in neutralising hCMV" is meant that an antibody molecule of the invention neutralises hCMV in a standard assay at a concentration much lower than antibodies known in the art, for example compared to MSL-109, 8F9 or 3E3.

In one embodiment, the antibody molecule of the present invention can neutralise hCMV at a concentration of 0.16µg/ml or lower (*i.e.* 0.15, 0.125, 0.1, 0.075, 0.05, 0.025, 0.02, 0.015, 0.0125, 0.01 , 0.0075, 0.005, 0.004, 0.003, 0.002 or lower). In another embodiment, the antibody can neutralise hCMV at a concentration of 0.016µg/ml or lower *(i.e.* at an antibody concentration lower than, for example, 10⁻⁸ M, 10⁻⁹ M, 10⁻¹⁰ M, 10⁻¹¹ M, 10⁻¹² M, 10⁻¹³ M). This means that only very low concentrations of antibody are required for 50% neutralisation of a clinical isolate of hCMV *in vitro* compared to the concentration of known antibodies, *e.g.,* MSL-109, 8F9 or 3E3, required for neutralisation of the same titre of hCMV. In a further embodiment, the concentration of antibody of the invention required for 50% neutralisation of infection of endothelial cells, epithelial cells and dendritic cells by a clinical isolate of hCMV in vitro is 10 times or more *(i.e.* 25, 50, 75, 100, 150, 200 or more) lower than that required by MSL-109, 8F9 or 3E3. Potency can be measured using a standard neutralisation assay as described in the art.

The antibodies of the invention are able to neutralise hCMV infection of several cell types. An antibody according to the invention prevents infection of endothelial cells, retinal cells, and dendritic cells.

These antibodies can be used as prophylactic or therapeutic agents upon appropriate formulation, or as a diagnostic tool.

A "neutralising antibody" is one that can neutralise the ability of that pathogen to initiate and/or perpetuate an infection in a host. The invention provides a neutralising human monoclonal antibody, wherein the antibody recognises an antigen from hCMV.

Specifically, an antibody according to the invention has specificity for a combination of the hCMV proteins UL128, UL130 and UL131A.

In one embodiment, an antibody according to the invention is a monoclonal antibody referred to herein as 6G4. This antibody, isolated from a hCMV infected donor, is produced by the immortalised B cell clone referred to as 6G4. This antibody neutralises hCMV infection of endothelial cells, epithelial cells, such as retinal cells, and myeloid cells, such as dendritic cells.

The heavy chain of 6G4 has the amino acid sequence recited in SEQ ID NO: 7 and the light chain has the amino acid sequence recited in SEQ ID NO: 8. The CDRs of the antibody heavy chains are referred to as CDRH1, CDRH2 and CDRH3, respectively. Similarly, the CDRs of the antibody light chains are referred to as CDRL1, CDRL2 and CDRL3, respectively. The position of the CDR amino acids are defined according to the IMGT numbering system [12, 13, 14] as: CDR1 - IMGT positions 27 to 38, CDR2 - IMGT positions 56 to 65 and CDR3 - IMGT positions 105 to 117.

The amino acid sequences of the CDRs of this antibody are shown in Table 1.

**Table 1**

| | **6G4** |
|---|---|
| **CDRH1** | GYRFTSYY (SEQ ID NO: 1) |
| **CDRH2** | IYPGDSDI (SEQ ID NO: 2) |
| **CDRH3** | ARLSLTESGDYVGAFDI (SEQ ID NO: 3) |
| **CDRL1** | QSLYYSDDNIF (SEQ ID NO: 4) |
| **CDRL2** | KVS (SEQ ID NO: 5) |
| **CDRL3** | MQGRHWPPLFT (SEQ ID NO: 6) |

The antibodies of the invention comprise a heavy chain comprising all three heavy chain CDRs from 6G4 (SEQ ID NOs: 1-3). That is, an antibody according to the invention comprises a heavy chain comprising (i) SEQ ID NO: 1 for CDRH1, SEQ ID NO: 2 for CDRH2 and SEQ ID NO: 3 for CDRH3.

The antibodies of the invention comprisea light chain comprising all three light chain CDRs from 6G4 (SEQ ID NOs: 4-6). That is, an antibody of the invention comprisesa light chain comprising (i) SEQ ID NO: 4 for CDRL1, SEQ ID NO: 5 for CDRL2 and SEQ ID NO: 6 for CDRL3.

An antibody according to the invention has specificity for a combination of the hCMV proteins UL 128, UL 130 and UL 131A and may comprise a heavy chain having an amino acid sequence that is at least 70%, at least 75%, at least 80%, at least 85%, at least 90%, at least 95%, at least 98%, or at least 99% identical to the amino acid sequence of SEQ ID NO: 7. In one embodiment, an antibody according to the invention comprises a heavy chain having the sequence recited in SEQ ID NO: 7.

An antibody according to the invention has specificity for a combination of the hCMV proteins UL128, UL130 and UL131A and may comprise a light chain having an amino acid sequence that is at least 70%, at least 75%, at least 80%, at least 85%, at least 90%, at least 95%, at least 98%, or at least 99% identical to the amino acid sequence of SEQ ID NO: 8. In yet another embodiment an antibody according to the invention comprises a light chain having the sequence recited in SEQ ID NO: 8.

In another aspect, the invention also includes nucleic acid sequences encoding antibodies and fragments thereof of the invention. Also described are nucleic acid sequences encoding part or all of the light and heavy chains and CDRs of the antibodies of the present invention. In one embodiment, nucleic acid sequences according to the invention include nucleic acid sequences having at least 70%, at least 75%, at least 80%, at least 85%, at least 90%, at least 95%, at least 98%, or at least 99% identity to the nucleic acid sequence of SEQ ID NO: 9 or SEQ ID NO: 10. A nucleic acid sequence of the invention may comprise the sequence of SEQ ID NO: 9 (encoding the 6G4 heavy chain variable region) or SEQ ID NO: 10 (encoding the 6G4 light chain variable region). Nucleic acid sequences of the invention may include those encoding the various CDRs include SEQ ID NO: 11 (encoding 6G4 CDRH 1), SEQ ID NO: 12 (encoding 6G4 CDRH2), SEQ ID NO: 13 (encoding 6G4 CDRH3), SEQ ID NO: 14 (encoding 6G4 CDRL1), SEQ ID NO: 15 (encoding 6G4 CDRL2) and SEQ ID NO: 16 (encoding 6G4 CDRL3). Due to the redundancy of the genetic code, variants of these sequences will exist that encode the same amino acid sequences. These variants are included within the scope of the invention.

Variant antibodies are also included within the scope of the invention. Thus, variants of the sequences recited in the application are also included within the scope of the invention. Such variants include natural variant generated by somatic mutation in vivo during the immune response or *in vitro* upon culture of immortalized B cell clones. Alternatively, variants may arise due to the degeneracy of the genetic code, as mentioned above. Alternatively, natural variants may be produced due to errors in transcription or translation.

Further variants of the antibody sequences having improved affinity and/or improved potency may be obtained using methods known in the art and are included within the scope of the invention. For example, amino acid substitutions may be used to obtain antibodies with further improved affinity.

Alternatively, codon optimisation of the nucleotide sequence may be used to improve the efficiency of translation in expression systems for the production of the antibody. Further, polynucleotides comprising a sequence optimized for antibody specificity or neutralising activity by the application of a directed evolution method to any of the nucleic acid sequences of the invention are also within the scope of the invention. In one embodiment variant antibody sequences may share 70% or more (i.e. 75%, 80%, 85%, 90%, 95%, 97%, 98%, 99% or more) amino acid sequence identity with the sequences recited in the application. In some embodiments such sequence identity is calculated with regard to the full length of the reference sequence (i.e. the sequence recited in the application). In some further embodiments, percentage identity, as referred to herein, is as determined using BLAST version 2.1.3 using the default parameters specified by the NCBI (the National Center for Biotechnology Information; http://www.ncbi.nlm.nih.gov/) [Blosum 62 matrix; gap open penalty=11 and gap extension penalty=1].

Further included within the scope of the invention are vectors, for example expression vectors, comprising a nucleic acid sequence according to the invention. Cells transformed with such vectors are also included within the scope of the invention. Examples of such cells include but are not limited to, eukaryotic cells, e.g. yeast cells, animal cells or plant cells. In one embodiment the cells are mammalian, e.g. human, CHO, HEK293T, PER.C6, NSO, myeloma or hybridoma cells.

Monoclonal and recombinant antibodies are particularly useful in identification and purification of the individual polypeptides or other antigens against which they are directed. The antibodies of the invention have additional utility in that they may be employed as reagents in immunoassays, radioimmunoassays (RIA) or enzyme-linked immunosorbent assays (ELISA). In these applications, the antibodies can be labelled with an analytically-detectable reagent such as a radioisotope, a fluorescent molecule or an enzyme. The antibodies may also be used for the molecular identification and characterisation (epitope mapping) of antigens.

Antibodies of the invention can be coupled to a drug for delivery to a treatment site or coupled to a detectable label to facilitate imaging of a site comprising cells of interest, such as cells infected with hCMV. Methods for coupling antibodies to drugs and detectable labels are well known in the art, as are methods for imaging using detectable labels. Labelled antibodies may be employed in a wide variety of assays, employing a wide variety of labels. Detection of the formation of an antibody- antigen complex between an antibody of the invention and an epitope of interest (a hCMV epitope) can be facilitated by attaching a detectable substance to the antibody. Suitable detection means include the use of labels such as radionuclides, enzymes, coenzymes, fluorescers, chemiluminescers, chromogens, enzyme substrates or co-factors, enzyme inhibitors, prosthetic group complexes, free radicals, particles, dyes, and the like. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material is luminol; examples of bioluminescent materials include luciferase, luciferin, and aequorin; and examples of suitable radioactive material include ¹²⁵I, ¹³¹I, ³⁵S, or ³H. Such labeled reagents may be used in a variety of well-known assays, such as radioimmunoassays, enzyme immunoassays, e.g., ELISA, fluorescent immunoassays, and the like. See for example, references 15-18.

An antibody according to the invention may be conjugated to a therapeutic moiety such as a cytotoxin, a therapeutic agent, or a radioactive metal ion or radioisotope. Examples of radioisotopes include, but are not limited to, I-131, I-123, I-125, Y-90, Re-188, Re-186, At-211, Cu-67, Bi-212, Bi-213, Pd-109, Tc-99, In-111, and the like. Such antibody conjugates can be used for modifying a given biological response; the drug moiety is not to be construed as limited.to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin.

Techniques for conjugating such therapeutic moiety to antibodies are well known. See, for example, Arnon et al. (1985) "Monoclonal Antibodies for Immunotargeting of Drugs in Cancer Therapy," in Monoclonal Antibodies and Cancer Therapy, ed. Reisfeld et al. (Alan R. Liss, Inc.), pp. 243-256; ed. Hellstrom et al. (1987) "Antibodies for Drug Delivery," in Controlled Drug Delivery, ed. Robinson et al. (2d ed; Marcel Dekker, Inc.), pp. 623-653; Thorpe (1985) "Antibody Carriers of Cytotoxic Agents in Cancer Therapy: A Review," in Monoclonal Antibodies '84: Biological and Clinical Applications, ed. Pinchera et al. pp. 475-506 (Editrice Kurds, Milano, Italy, 1985); "Analysis, Results, and Future Prospective of the Therapeutic Use of Radiolabeled Antibody in Cancer Therapy," in Monoclonal Antibodies for Cancer Detection and Therapy, ed. Baldwin et al. (Academic Press, New York, 1985), pp. 303-316; and Thorpe et al. (1982) Immunol. Rev. 62: 1 19- 158.

Alternatively, an antibody can be conjugated to a second antibody to form an antibody heteroconjugate as described in reference 19. In addition, linkers may be used between the labels and the antibodies of the invention [20]. Antibodies or antigen-binding fragments thereof may be directly labelled with radioactive iodine, indium, yttrium, or other radioactive particle known in the art [21]. Treatment may consist of a combination of treatment with conjugated and non-conjugated antibodies administered simultaneously or subsequently [22, 23].

Antibodies of the invention may be attached to a solid support.

Additionally, antibodies of the invention, or functional antibody fragments thereof, can be chemically modified by covalent conjugation to a polymer, for example, to increase their circulating half-life. Examples of polymers, and methods to attach them to peptides, are shown in references 24-27. In some embodiments the polymers may be selected from polyoxyethylated polyols and polyethylene glycol (PEG). PEG is soluble in water at room temperature and has the general formula: R(O--CH₂ --CH₂)ₙ O--R where R can be hydrogen, or a protective group such as an alkyl or alkanol group. In one embodiment the protective group may have between 1 and 8 carbons. In a further embodiment the protective group is methyl. The symbol n is a positive integer. In one embodiment n is between 1 and 1,000. In another embodiment n is between 2 and 500. In one embodiment the PEG has an average molecular weight between 1,000 and 40,000. In a further embodiment the PEG has a molecular weight between 2,000 and 20,000. In yet a further embodiment the PEG has a molecular weight of between 3,000 and 12,000. In one embodiment PEG has at least one hydroxy group. In another embodiment the PEG has a terminal hydroxy group. In yet another embodiment it is the terminal hydroxy group which is activated to react with a free amino group on the inhibitor. However, it will be understood that the type and amount of the reactive groups may be varied to achieve a covalently conjugated PEG/antibody of the present invention.

Water-soluble polyoxyethylated polyols are also useful in the present invention. They include polyoxyethylated sorbitol, polyoxyethylated glucose, polyoxyethylated glycerol (POG), and the like. In one embodiment, POG is used. Without being bound by any theory, this may be because the glycerol backbone of polyoxyethylated glycerol is the same backbone occurring naturally in, for example, animals and humans in mono-, di-, triglycerides, this branching would therefore not necessarily be seen as a foreign agent in the body. In some embodiments POG has a molecular weight in the same range as PEG. The structure for POG is shown in reference 28.

Another drug delivery system that can be used for increasing circulatory half-life is the liposome. Methods of preparing liposome delivery systems are discussed in references 29, 30 and 31. Other drug delivery systems are known in the art and are described in, for example, references 32 and 33.

Antibodies of the invention may be provided in purified form. Typically, the antibody will be present in a composition that is substantially free of other polypeptides *e*.*g*. where less than 90% (by weight), usually less than 60% and more usually less than 50% of the composition is made up of other polypeptides.

Antibodies of the invention may be immunogenic in non-human (or heterologous) hosts e.g. in mice. In particular, the antibodies may have an idiotope that is immunogenic in non-human hosts, but not in a human host. Antibodies of the invention for human use include those that cannot be easily isolated from hosts such as mice, goats, rabbits, rats, non-primate mammals, *etc.* and cannot generally be obtained by humanisation or from xeno-mice.

Antibodies of the invention can be of any isotype *(e.g.* IgA, IgG, IgM *i.e.* an α, γ or µ heavy chain), but will generally be IgG. Within the IgG isotype, antibodies may be IgG1, IgG2, IgG3 or IgG4 subclass. Antibodies of the invention may have a κ or a λ light chain.

### Production of antibodies

Monoclonal antibodies according to the invention can be made by any method known in the art. The general methodology for making monoclonal antibodies using hybridoma technology is well known [34,35]. Preferably, the alternative EBV immortalisation method described in reference 36 is used.

Using the method described in reference 36, B cells producing the antibody of the invention can be transformed with EBV in the presence of a polyclonal B cell activator. Transformation with EBV is a standard technique and can easily be adapted to include polyclonal B cell activators.

Additional stimulants of cellular growth and differentiation may optionally be added during the transformation step to further enhance the efficiency. These stimulants may be cytokines such as IL-2 and IL-15. In one aspect, IL-2 is added during the immortalisation step to further improve the efficiency of immortalisation, but its use is not essential.

The immortalised B cells produced using these methods can then be cultured using methods known in the art and antibodies isolated therefrom.

Monoclonal antibodies may be further purified, if desired, using filtration, centrifugation and various chromatographic methods such as HPLC or affinity chromatography. Techniques for purification of monoclonal antibodies, including techniques for producing pharmaceutical-grade antibodies, are well known in the art.

Fragments of the monoclonal antibodies of the invention can be obtained from the monoclonal antibodies by methods that include digestion with enzymes, such as pepsin or papain, and/or by cleavage of disulfide bonds by chemical reduction. Alternatively, fragments of the monoclonal antibodies can be obtained by cloning and expression of part of the sequences of the heavy or light chains. Antibody "fragments" may include Fab, Fab', F(ab')₂ and Fv fragments. The invention also encompasses single-chain Fv fragments (scFv) derived from the heavy and light chains of a monoclonal antibody of the invention e.g. the invention includes a scFv comprising the CDRs from an antibody of the invention. Also included are heavy or light chain monomers and dimers as well as single chain antibodies, *e*.*g*. single chain Fv in which the heavy and light chain variable domains are joined by a peptide linker.

Standard techniques of molecular biology may be used to prepare DNA sequences coding for the antibodies or fragments of the antibodies of the present invention. Desired DNA sequences may be synthesised completely or in part using oligonucleotide synthesis techniques. Site-directed mutagenesis and polymerase chain reaction (PCR) techniques may be used as appropriate.

Any suitable host cell/vector system may be used for expression of the DNA sequences encoding the antibody molecules of the present invention or fragments thereof. Bacterial, for example *E. coli,* and other microbial systems may be used, in part, for expression of antibody fragments such as Fab and F(ab')₂ fragments, and especially Fv fragments and single chain antibody fragments, for example, single chain Fvs. Eukaryotic, e.g. mammalian, host cell expression systems may be used for production of larger antibody molecules, including complete antibody molecules. Suitable mammalian host cells include CHO, HEK293T, PER.C6, NSO, myeloma or hybridoma cells.

A process for the production of an antibody molecule according to the present invention may comprise culturing a host cell comprising a vector of the present invention under conditions suitable for leading to expression of protein from DNA encoding the antibody molecule of the present invention, and isolating the antibody molecule.

The antibody molecule may comprise only a heavy or light chain polypeptide, in which case only a heavy chain or light chain polypeptide coding sequence needs to be used to transfect the host cells. For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides.

Alternatively, antibodies according to the invention may be produced by i) expressing a nucleic acid sequence according to the invention in a cell, and ii) isolating the expressed antibody product. Additionally, the method may include iii) purifying the antibody.

### Screening and isolation of B cells

Transformed B cells may be screened for those producing antibodies of the desired antigen specificity, and individual B cell clones may then be produced from the positive cells.

The screening step may be carried out by ELISA, by staining of tissues or cells (including transfected cells), a neutralisation assay or one of a number of other methods known in the art for identifying desired antigen specificity. The assay may select on the basis of simple antigen recognition, or may select on the additional basis of a desired function e.g. to select neutralising antibodies rather than just antigen-binding antibodies, to select antibodies that can change characteristics of targeted cells, such as their signalling cascades, their shape, their growth rate, their capability of influencing other cells, their response to the influence by other cells or by other reagents or by a change in conditions, their differentiation status, *etc.*

The cloning step for separating individual clones from the mixture of positive cells may be carried out using limiting dilution, micromanipulation, single cell deposition by cell sorting or another method known in the art.

These immortalised B cell clones can be used in various ways *e.g.* as a source of monoclonal antibodies, as a source of nucleic acid (DNA or mRNA) encoding a monoclonal antibody of interest, for research, etc.

A composition may comprise immortalised B memory lymphocytes, wherein the lymphocytes produce antibodies with high neutralising potency specific for hCMV, and wherein the antibodies are produced at ≥5pg per cell per day. A composition may comprise clones of an immortalised B memory lymphocyte, wherein the clones produce a monoclonal antibody with high neutralising potency specific for hCMV, and wherein the antibody is produced at ≥5pg per cell per day. Preferably said clones produce a monoclonal antibody with a high potency in neutralizing hCMV infection. The preferred immortalised B cell clone is 6G4.

### Epitopes

As mentioned above, the antibodies of the invention can be used to map the epitopes to which they bind. The inventors have discovered that the antibody 6G4 which neutralises hCMV infection of endothelial cells, epithelial cells, such as retinal cells, and myeloid cells, such as dendritic cells, is directed towards an epitope determined by a combination of the hCMV proteins UL128, UL130 and UL131A. Although the inventors do not wish to be bound by this theory, it is believed that the antibody 6G4 binds to a conformational epitope formed by these three proteins.

The epitope recognised by the antibodies of the present invention may have a number of uses. The epitope and mimotopes thereof in purified or synthetic form can be used to raise immune responses (*i.e.* as a vaccine, or for the production of antibodies for other uses) or for screening patient serum for antibodies that immunoreact with the epitope or mimotopes thereof. Such an epitope or mimotope, or antigen comprising such an epitope or mimotope may be used as a vaccine for raising an immune response. The antibodies and antibody fragments of the invention can also be used in a method of monitoring the quality of vaccines. In particular the antibodies can be used to check that the antigen in a vaccine contains the correct immunogenic epitope in the correct conformation.

The epitope may also be useful in screening for ligands that bind to said epitope. Such ligands, include but are not limited to antibodies; including those from camels, sharks and other species, fragments of antibodies, peptides, phage display technology products, aptamers, adnectins or fragments of other viral or cellular proteins, may block the epitope and so prevent infection.

### Recombinant expression

The immortalised memory B lymphocytes described herein may also be used as a source of nucleic acid for the cloning of antibody genes for subsequent recombinant expression. Expression from recombinant sources is more common for pharmaceutical purposes than expression from B cells or,hybridomas *e.g.* for reasons of stability, reproducibility, culture ease, etc.

Thus a method for preparing a recombinant cell, may comprise the steps of: (i) obtaining one or more nucleic acids (*e.g*. heavy and/or light chain genes) from the B cell clone that encodes the antibody of interest; and (ii) inserting the nucleic acid into an expression host in order to permit expression of the antibody of interest in that host.

Similarly, a method for preparing a recombinant cell, may comprise the steps of: (i) sequencing nucleic acid(s) from the B cell clone that encodes the antibody of interest; and (ii) using the sequence information from step (i) to prepare nucleic acid(s) for insertion into an expression host in order to permit expression of the antibody of interest in that host. The nucleic acid may, but need not, be manipulated between steps (i) and (ii) to introduce restriction sites, to change codon usage, and/or to optimise transcription and/or translation regulatory sequences.

A method of preparing a recombinant cell, may comprise the step of transforming a host cell with one or more nucleic acids that encode a monoclonal antibody of interest, wherein the nucleic acids are nucleic acids that were derived from an immortalised B cell clone described herein. Thus the procedures for first preparing the nucleic acid(s) and then using it to transform a host cell can be performed at different times by different people in different places (*e.g*. in different countries).

These recombinant cells can then be used for expression and culture purposes. They are particularly useful for expression of antibodies for large-scale pharmaceutical production. They can also be used as the active ingredient of a pharmaceutical composition. Any suitable culture techniques can be used, including but not limited to static culture, roller bottle culture, ascites fluid, hollow-fiber type bioreactor cartridge, modular minifermenter, stirred tank, microcarrier culture, ceramic core perfusion, etc.

Methods for obtaining and sequencing immunoglobulin genes from B cells are well known in the art *(e.g.* see reference 37).

The expression host is preferably a eukaryotic cell, including yeast and animal cells, particularly mammalian cells (*e.g*. CHO cells, NSO cells, human cells such as PER.C6 [Crucell; reference 38] or HKB-11 [Bayer; references 39 & 40] cells, myeloma cells [41 & 42], etc.), as well as plant cells. Preferred expression hosts can glycosylate the antibody of the invention, particularly with carbohydrate structures that are not themselves immunogenic in humans. The expression host may be able to grow in serum-free media. The expression host may be able to grow in culture without the presence of animal-derived products.

The expression host may be cultured to give a cell line.

A method for preparing one or more nucleic acid molecules (*e.g*. heavy and light chain genes) that encode an antibody of interest, may comprise the steps of: (i) preparing an immortalised B cell clone as described herein; and (ii) obtaining from the B cell clone nucleic acid that encodes the antibody of interest. A method for obtaining a nucleic acid sequence that encodes an antibody of interest, may comprise the steps of: (i) preparing an immortalised B cell clone as described herein; and (ii) sequencing nucleic acid from the B cell clone that encodes the antibody of interest.

A method of preparing nucleic acid molecule(s) that encodes an antibody of interest, may comprise the step of obtaining the nucleic acid from a B cell clone that was obtained from a transformed B cell described herein. Thus the procedures for first obtaining the B cell clone and then preparing nucleic acid(s) from it can be performed at very different times by different people in different places (*e.g*. in different countries).

A method for preparing an antibody (*e.g.* for pharmaceutical use), may comprise the steps of: (i) obtaining and/or sequencing one or more nucleic acids (*e.g*. heavy and light chain genes) from the selected B cell clone expressing the antibody of interest; (ii) inserting the nucleic acid(s) into or using the nucleic acid(s) to prepare an expression host that can express the antibody of interest; (iii) culturing or sub-culturing the expression host under conditions where the antibody of interest is expressed; and, optionally, and (iv) purifying the antibody of the interest.

A method of preparing an antibody may comprise the steps of: culturing or sub-culturing an expression host cell population under conditions where the antibody of interest is expressed and, optionally, purifying the antibody of the interest, wherein said expression host cell population has been prepared by (i) providing nucleic acid(s) encoding a selected B cell the antibody of interest that is produced by a population of B memory lymphocytes prepared as described above, (ii) inserting the nucleic acid(s) into an expression host that can express the antibody of interest, and (iii) culturing or sub-culturing expression hosts comprising said inserted nucleic acids to produce said expression host cell population. Thus the procedures for first preparing the recombinant expression host and then culturing it to express antibody can be performed at very different times by different people in different places (*e.g.* in different countries).

### Pharmaceutical compositions

The invention provides a pharmaceutical composition containing the antibodies and/or antibody fragments of the invention. Also described are pharmaceutical compositions containing nucleic acid encoding such antibodies and/or immortalised B cells that express such antibodies and/or the epitopes recognised by the antibodies of the invention. A pharmaceutical composition may also contain a pharmaceutically acceptable carrier to allow administration. The carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Within the scope of the invention, forms of administration may include those forms suitable for parenteral administration, *e.g*. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the antibody molecule may be in dry form, for reconstitution before use with an appropriate sterile liquid.

Once formulated, the compositions of the invention can be administered directly to the subject. In one embodiment the compositions are adapted for administration to human subjects.

The pharmaceutical compositions of this invention may be administered by any number of routes including, but not limited to, oral, intravenous, intramuscular, intra-arterial, intramedullary, intraperitoneal, intrathecal, intraventricular, transdermal, transcutaneous, topical, subcutaneous, intranasal, enteral, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue.

The compositions can also be administered into a lesion. Dosage treatment may be a single dose schedule or a multiple dose schedule. Known antibody-based pharmaceuticals provide guidance relating to frequency of administration e.g. whether a pharmaceutical should be delivered daily, weekly, monthly, etc. Frequency and dosage may also depend on the severity of symptoms.

Compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g*. a lyophilised composition, like Synagis™ and Herceptin™, for reconstitution with sterile water containing a preservative). The composition may be prepared for topical administration *e.g.* as an ointment, cream or powder. The composition may be prepared for oral administration *e.g*. as a tablet or capsule, as a spray, or as a syrup (optionally flavoured). The composition may be prepared for pulmonary administration e.g. as an inhaler, using a fine powder or a spray. The composition may be prepared as a suppository or pessary. The composition may be prepared for nasal, aural or ocular administration *e.g.* as drops. The composition may be in kit form, designed such that a combined composition is reconstituted just prior to administration to a patient. For example, a lyophilised antibody can be provided in kit form with sterile water or a sterile buffer.

It will be appreciated that the active ingredient in the composition will be an antibody molecule, an antibody fragment or variants and derivatives thereof. As such, it will be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Gennaro (2000) Remington: The Science and Practice of Pharmacy, 20th edition, ISBN: 0683306472.

Pharmaceutical compositions of the invention generally have a pH between 5.5 and 8.5, in some embodiments this may be between 6 and 8, and in further embodiments about 7. The pH may be maintained by the use of a buffer. The composition may be sterile and/or pyrogen free. The composition may be isotonic with respect to humans. In one embodiment pharmaceutical compositions of the invention are supplied in hermetically-sealed containers.

Pharmaceutical compositions will include an effective amount of one or more antibodies of the invention and/or one or more immortalised B cells as described herein and/or a polypeptide comprising an epitope that binds an antibody of the invention *i*.*e*. an amount that is sufficient to treat, ameliorate, or prevent a desired disease or condition, or to exhibit a detectable therapeutic effect. Therapeutic effects also include reduction in physical symptoms. The precise effective amount for any particular subject will depend upon their size and health, the nature and extent of the condition, and the therapeutics or combination of therapeutics selected for administration. The effective amount for a given situation is determined by routine experimentation and is within the judgment of a clinician. For purposes of the present invention, an effective dose will generally be from about 0.01mg/kg to about 50mg/kg, or about 0.05mg/kg to about 10mg/kg of the compositions of the present invention in the individual to which it is administered. Known antibody-based pharmaceuticals provide guidance in this respect e.g. Herceptin™ is administered by intravenous infusion of a 21mg/ml solution, with an initial loading dose of 4mg/kg body weight and a weekly maintenance dose of 2mg/kg body weight; Rituxan™ is administered weekly at 375mg/m²; *etc.*

In one embodiment compositions can include more than one *(e.g.* 2, 3, 4, 5, *etc.)* antibodies of the invention to provide an additive or synergistic therapeutic effect. In a further embodiment the composition may comprise one or more *(e.g.* 2, 3, 4, 5, *etc.*) antibodies or antibody fragments of the invention and one or more *(e.g.* 2, 3, 4, 5, *etc.*) further antibodies or antibody fragments against hCMV. For example, one antibody may bind to the epitope determined by a combination of the hCMV proteins UL128, UL130 and UL131A while another may bind to a further hCMV protein. Within this embodiment the hCMV protein may be gB, gH, gL, gM, gN, gO, UL128, UL130 or UL131A, or a combination thereof. In a further embodiment, the second antibody or antibody fragment may be specific for the epitope which is recognised by MSL-109, 8F9 or 3E3. In yet a further embodiment, one antibody may be targeted to the mechanism that mediates infection of fibroblasts, while the other antibody may be targeted to the mechanism that mediates infection of endothelial cells. For optimal clinical effect it may well be advantageous to address both mechanisms of hCMV infection and maintenance.

Antibodies or antibody fragments of the invention may be administered (either combined or separately) with other therapeutics *e.g.* with chemotherapeutic compounds, with radiotherapy, *etc.* Preferred therapeutic compounds include anti-viral compounds such as ganciclovir, foscarnet and cidofovir. Such combination therapy provides an additive or synergistic improvement in therapeutic efficacy relative to the individual therapeutic agents when administered alone. The term "synergy" is used to describe a combined effect of two or more active agents that is greater than the sum of the individual effects of each respective active agent. Thus, where the combined effect of two or more agents results in "synergistic inhibition" of an activity or process, it is intended that the inhibition of the activity or process is greater than the sum of the inhibitory effects of each respective active agent. The term "synergistic therapeutic effect" refers to a therapeutic effect observed with a combination of two or more therapies wherein the therapeutic effect (as measured by any of a number of parameters) is greater than the sum of the individual therapeutic effects observed with the respective individual therapies.

Antibodies or antibody fragments may be administered to those patients who have previously shown no response to treatment for hCMV infection, *i*.*e*. have been shown to be refractive to anti-hCMV treatment. Such treatment may include previous treatment with an anti-viral agent. This may be due to, for example, infection with an anti-viral resistant strain of hCMV.

In compositions of the invention that include antibodies of the invention, the antibodies may make up at least 50% by weight (e.g. 60%, 70%, 75%, 80%, 85%, 90%, 95%, 97%, 98%, 99% or more) of the total protein in the composition. The antibodies are thus in purified form.

A method of preparing a pharmaceutical, may comprise the steps of: (i) preparing an antibody of the invention; and (ii) admixing the purified antibody with one or more pharmaceutically-acceptable carriers.

A method of preparing a pharmaceutical, may comprise the step of admixing an antibody with one or more pharmaceutically-acceptable carriers, wherein the antibody is a monoclonal antibody that was obtained from a transformed B cell described herein. Thus the procedures for first obtaining the monoclonal antibody and then preparing the pharmaceutical can be performed at very different times by different people in different places (e.g. in different countries).

As an alternative to delivering antibodies or B cells for therapeutic purposes, it is possible to deliver nucleic acid (typically DNA) that encodes the monoclonal antibody (or active fragment thereof) of interest to a subject, such that the nucleic acid can be expressed in the subject *in situ* to provide a desired therapeutic effect. Suitable gene therapy and nucleic acid delivery vectors are known in the art.

Compositions may include an antimicrobial, particularly if packaged in a multiple dose format.

Compositions may comprise detergent e.g. a Tween (polysorbate), such as Tween 80. Detergents are generally present at low levels e.g. <0.01%.

Compositions may include sodium salts (e.g. sodium chloride) to give tonicity. A concentration of 10±2mg/ml NaCl is typical.

Compositions may comprise a sugar alcohol *(e.g.* mannitol) or a disaccharide *(e.g.* sucrose or trehalose) *e.g.* at around 15-30mg/ml *(e.g.* 25mg/ml), particularly if they are to be lyophilised or if they include material which has been reconstituted from lyophilised material. The pH of a composition for lyophilisation may be adjusted to around 6.1 prior to lyophilisation.

The compositions of the invention may also comprise one or more immunoregulatory agents. In one embodiment, one or more of the immunoregulatory agents include(s) an adjuvant.

### Medical treatments and uses

The antibodies and antibody fragments of the invention or derivatives and variants thereof may be used for the treatment of hCMV infection, for the prevention of hCMV infection or for the diagnosis of hCMV infection.

Methods of diagnosis may include contacting an antibody or an antibody fragment with a sample. Such samples may be tissue samples taken from, for example, salivary glands, lung, liver, pancreas, kidney, ear, eye, placenta, alimentary tract, heart, ovaries, pituitary, adrenals, thyroid, brain or skin. The methods of diagnosis may also include the detection of an antigen/antibody complex.

The invention therefore provides an antibodyor an antibody fragment, thereof according to the invention, for use in therapy.

A method of treating a patient may comprise administering to that patient an antibody or an antibody fragment thereof according to the invention.

The invention also provides the use of an antibody or an antibody fragment thereof according to the invention in the manufacture of a medicament for the prevention or treatment of hCMV infection.

The invention provides a composition of the invention for use as a medicament for the prevention or treatment of a hCMV infection. It also provides the use of an antibody of the invention in the manufacture of a medicament for treatment of a patient and/or diagnosis in a patient. A method for treating a subject and/or of performing diagnosis on a subject, may comprise the step of administering to them a composition of the invention. In some embodiments the subject may be a human. One way of checking efficacy of therapeutic treatment involves monitoring disease symptoms after administration of the composition of the invention. Treatment can be a single dose schedule or a multiple dose schedule.

An antibody, antibody fragment, immortalised B cell clone, epitope or composition described herein may be administered to a subject in need of such treatment. Such a subject includes, but is not limited to, one who is particularly at risk of or susceptible to hCMV infection, including, for example, an immunocompromised subject. Exemplary subjects include those suffering from HIV or undergoing immunosuppressive therapy, such as transplant patients.

Antibodies of the invention can be used in passive immunisation.

Antibodies and fragments thereof as described in the present invention may also be used in a kit for the diagnosis of hCMV infection.

Epitopes capable of binding an antibody of the invention, *e.g*., the monoclonal antibody 6G4, may be used in a kit for monitoring the efficacy of vaccination procedures by detecting the presence of protective anti-hCMV antibodies.

Antibodies, antibody fragments, or variants and derivatives thereof, as described in the present invention may also be used in a kit for monitoring vaccine manufacture with the desired immunogenicity.

A method of preparing a pharmaceutical, may comprise the step of admixing a monoclonal antibody with one or more pharmaceutically-acceptable carriers, wherein the monoclonal antibody is a monoclonal antibody that was obtained from an expression host of the invention. Thus the procedures for first obtaining the monoclonal antibody (*e.g*. expressing it and/or purifying it) and then admixing it with the pharmaceutical carrier(s) can be performed at very different times by different people in different places (*e.g*. in different countries).

Starting with a transformed B cell described herein, various steps of culturing, sub-culturing, cloning, sub-cloning, sequencing, nucleic acid preparation *etc.* can be performed in order to perpetuate the antibody expressed by the transformed B cell, with optional optimisation at each step. Preferably, the above methods further comprise techniques of optimisation (*e.g*. affinity maturation or optimisation) applied to the nucleic acids encoding the antibody.

In all these methods, the nucleic acid used in the expression host may be manipulated to insert, delete or amend certain nucleic acid sequences. Changes from such manipulation include, but are not limited to, changes to introduce restriction sites, to amend codon usage, to add or optimise transcription and/or translation regulatory sequences, *etc.* It is also possible to change the nucleic acid to alter the encoded amino acids. For example, it may be useful to introduce one or more (*e.g.* 1, 2, 3, 4, 5, 6, 7, 8, 9, 10, etc.) amino acid substitutions, deletions and/or insertions into the antibody's amino acid sequence. Such point mutations can modify effector functions, antigen-binding affinity, post-translational modifications, immunogenicity, *etc.,* can introduce amino acids for the attachment of covalent groups (*e.g*. labels) or can introduce tags *(e.g.* for purification purposes). Mutations can be introduced in specific sites or can be introduced at random, followed by selection (*e.g*. molecular evolution).

### General

The term "comprising" encompasses "including" as well as "consisting" *e.g*. a composition "comprising" X may consist exclusively of X or may include something additional e.g. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value *x* means, for example, *x*±10%.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

As used herein, reference to "treatment" of a patient is intended to include prevention and prophylaxis. The term "patient" means all mammals including humans. Examples of patients include humans, cows, dogs, cats, horses, goats, sheep, pigs, and rabbits. Generally, the patient is a human.

### Examples

The following examples are provided by way of illustration only, and are not intended to be limiting.

### Example 1: Cloning of B cells and screening for hCMV neutralising activity

A donor with high hCMV neutralising antibody titres in the serum was identified. Memory B cells were isolated and immortalised using EBV and CpG as described in reference 36. Briefly, memory B cells were isolated by negative selection using CD22 beads, followed by removal of IgM⁺, IgD⁺ IgA⁺ B cells using specific antibodies and cell sorting. The sorted cells (IgG⁺) were immortalised with EBV in the presence of CpG 2006 and irradiated allogeneic mononuclear cells. Replicate cultures each containing 50 memory B cells were set up in twenty 96 well U bottom plates. After two weeks the culture supernatants were collected and tested for their capacity to neutralise hCMV infection of either fibroblasts or epithelial cells in separate assays. B cell clones were isolated from positive polyclonal cultures as described in reference 36. IgG concentrations in the supernatant of selected clones were determined using an IgG-specific ELISA.

For the viral neutralisation assay a titrated amount of a clinical hCMV isolate was mixed with an equal volume of culture supernatant or with dilutions of human sera containing neutralising antibodies. After 1 hour incubation at room temperature the mixture was added to confluent monolayers of either endothelial cells (*e.g*. HMEC-1 cells), epithelial cells (*e.g.* ARPE retinal cells) or fibroblasts (*e.g*. MRC-9 or mesenchymal stem cells) in 96 well flat bottom plates and incubated at 37°C for two days. The supernatant was discarded, the cells were fixed with cold methanol and stained with a mixture of mouse monoclonal antibodies to hCMV early antigens, followed by a fluorescein-labelled goat anti mouse Ig. The plates were analyzed using a fluorescence microscope. In the absence of neutralising antibodies the infected cells were ∼1,000/field, while in the presence of saturating concentrations of neutralising antibodies the infection was completely inhibited. The viral neutralization assay was also performed using dendritic cells as target cells. The neutralising titer is indicated as the concentration of antibody (µg/ml) that gives a 50% reduction of hCMV infection. Table 2 shows that 6G4, which has been shown to be specific for a combination of UL128, UL130 and UL131A, was able to neutralise hCMV infection of endothelial, retinal and dendritic cells at very low concentrations (i.e. with high potency).

**Table 2**

| | **50% Neutralisation (µg/ml) of:** | |
|---|---|---|
| **Clone** | **Fibroblasts** | **Endothelial/retinal/dendritic cells** |
| 6G4 | * | 0.004 |
| Cytotec^{□} | 5,000 | 50 |
| Donor's Serum | 33 | 1 |

| | | |
|---|---|---|
| *no neutralisation at the highest concentration tested (*i.e.* >2µg/ml). □Cytotect (Biotest) is a pool of hCMV hyperimmune IgG. | | |

### Example 2: Identification of the target antigens recognised by the monoclonal antibodies

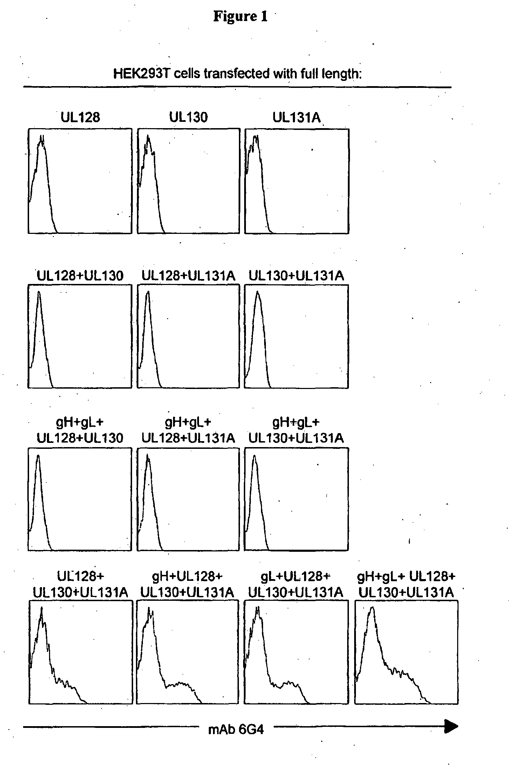

To map the specificities of the human monoclonal antibody 6G4 neutralizing infection of endothelial cells, expression vectors encoding full length UL128, UL130, UL131A, gH and gL were constructed. HEK293T cells were transfected with these vectors alone or in combination. After 36h, cells were fixed, permeabilized and stained with 6G4 followed by goat anti-human IgG. Figure 1 shows that monoclonal antibody 6G4 stained cells co-expressing at least UL128, UL130 and UL131A. The intensity of the 6G4 staining was increased when gH and gL were co-transfected together with UL128, UL130 and UL131A to reconstitute the putative whole glycoprotein complex gCIII. These data suggest that the monoclonal antibody 6G4 is specific for a conformational epitope determined by a combination of the hCMV proteins UL128, UL130 and UL131A. Most likely this epitope is in the proper conformation only when UL128, UL130 and UL131A are assembled in gCIII with gH and gL

### REFERENCES

[1] Plachter et al. (1996) Adv Virus Res 46:195-261.
[2] Gerna et al. (2002) J Med Virol 66:335-339.
[3] Adler et al. (2006) J Gen Virol 87:2451-2460.
[4] Gerna et al. (2005) J Gen Virol 86:275-284.
[5] Hahn et al. (2004) J Virol 78:10023-10033.
[6] Patrone et al. (2005) J Virol 79:8361-8373.
[7] Wang (2005) Proc Natl Acad Sci USA 102:18153-18158.
[8] Wang et al. (2005) J Virol 79:10330-10338.
[9] Nigro et al. (2005) N Engl J Med 353:1350-1362.
[10] Borucki et al. (2004) Antiviral Res 64:103-111.
[11] McLean et al. (2005) J Immunol, 174:4768-4778.
[12] Lefranc et al. (2003) Dev Comp Immunol. 27(1):55-77.
[13] Lefranc et al. (1997) Immunology Today, 18:509.
[14] Lefranc (1999) The Immunologist, 7:132-136.
[15] US 3,766,162
[16] US 3,791,932
[17] US 3,817,837
[18] US 4,233,402
[19] US 4,676,980
[20] US 4,831,175
[21] US 5,595,721
[22] WO00/52031
[23] WO00/52473
[24] US 4,766,106
[25] US 4,179,337
[26] US 4,495,285
[27] US 4,609,546
[28] Knauf et al. (1988) J. Bio. Chem. 263:15064-15070
[29] Gabizon et al. (1982) Cancer Research 42:4734
[30] Cafiso (1981) Biochem Biophys Acta 649:129
[31] Szoka (1980) Ann. Rev. Biophys. Eng. 9:467
[32] Poznansky et al. (1980) Drug Delivery Systems (R.L. Juliano, ed., Oxford, N.Y.) pp. 253-315
[33] Poznansky (1984) Pharm Revs 36:277
[34] Kohler, G. and Milstein, C,. 1975, Nature 256:495-497.
[35] Kozbar et al. 1983, Immunology Today 4:72*.*
[36] WO2004/076677
[37] Chapter 4 of Kuby Immunology (4th edition, 2000: ASIN: 0716733315
[38] Jones et al. Biotechnol Prog 2003, 19(1): 163-8
[39] Cho et al. Cytotechnology 2001,37:23-30
[40] Cho et al. Biotechnol Prog 2003,19:229-32
[41] US 5,807,715
[42] US 6,300,104
[43] WO 2007/146024
[44] WO 2008/084410

## Claims

1. An isolated antibody, or an antigen binding fragment thereof, that is specific for a combination of hCMV proteins UL128, UL130 and UL131A, the combination forming the epitope recognized by said antibody or binding fragment, and that neutralizes infection of endothelial cells, retinal cells, or dendritic cells by human cytomegalovirus (hCMV), wherein the antibody or fragment comprises CDR1, CDR2, and CDR3 heavy chain sequences as set forth in SEQ ID NO: 1, SEQ ID NO: 2, and SEQ ID NO: 3 respectively, and CDR1, CDR2, and CDR3 light chain sequences as set forth in SEQ ID NO: 4, SEQ ID NO: 5, and SEQ ID NO: 6 respectively.

2. The antibody or fragment of claim 1, comprising heavy and light chain variable regions as set forth in SEQ ID NO: 7 and SEQ ID NO: 8 respectively.

3. The antibody or fragment of claim 1 or 2, that binds a conformational epitope formed by the three proteins UL128, UL130 and UL131A.

4. The antibody or fragment of any one of claims 1-3, wherein the antibody is a monoclonal antibody, a human monoclonal antibody, a single chain antibody, Fab, Fab', F(ab')₂, Fv or scFv.

5. An isolated nucleic acid molecule comprising a nucleotide sequence encoding the antibody or fragment of any one of the preceding claims.

6. The nucleic acid molecule of claim 5 comprising the nucleotide sequence of any one of SEQ ID NOs: 9, 10, 11, 12, 13, 14, 15, or 16, or a nucleotide sequence encoding the same amino acid sequence as the nucleotide sequence of any one of SEQ ID NOs: 9, 10, 11, 12, 13, 14, 15 or 16.

7. An expression vector comprising a nucleic acid molecule according to claim 5 or 6.

8. A cell transformed with the vector according to claim 7.

9. A pharmaceutical composition comprising the antibody or fragment of any one of claims 1-4 and a pharmaceutically acceptable diluent or carrier.

10. The composition of claim 9 further comprising a second antibody against hCMV, or an antigen binding fragment thereof.

11. The composition of claim 9 further comprising
(a) a second antibody, or antigen binding fragment thereof, which binds to an hCMV protein selected from gB, gH, gL, gM, gN, gO, UL128, UL130 and UL131A, or a combination thereof; or
(b) a second antibody, or antigen binding fragment thereof, which binds to the hCMV protein gB.

12. Use of the antibody or fragment of any one of claims 1-4, the nucleic acid of claim 5 or 6, or the cell of claim 8, or the composition of any of claims 9 to 11, in the manufacture of a medicament for the prevention or treatment of hCMV infection.

13. The antibody or fragment of any one of claims 1-4 or the composition of any of claims 9 to 11, for use in the prevention or treatment of hCMV infection.

14. A composition comprising an antibody or fragment of any one of claims 1 to 4 for use in the prevention or treatment of hCMV infection, wherein the composition further comprises a second antibody selected from:
(a) an antibody or antigen binding fragment thereof against hCMV;
(b) an antibody or antigen binding fragment thereof which binds to an hCMV protein selected from gB, gH, gL, gM, gN, gO, UL128, UL130 and UL131A, or a combination thereof; and
(c) an antibody or antigen binding fragment thereof which binds to the hCMV protein gB.

15. A method for producing the antibody or fragment of any one of claims 1-4, comprising (i) culturing the cell of claim 8 and (ii) isolating the antibody or fragment.

## Patentansprüche

1. Isolierter Antikörper oder Antigen-bindendes Fragment davon, das für eine Kombination der hCMV-Proteine UL128, UL130 und UL131A spezifisch ist, wobei die Kombination das Epitop bildet, das durch den Antikörper oder das bindende Fragment erkannt wird und eine Infektion von Endothelzellen, retinalen Zellen oder dendritischen Zellen durch ein humanes Cytomegalovirus (hCMV) neutralisiert, wobei der Antikörper oder das Fragment Folgendes umfasst: CDR1-, CDR2- und CDR3- Schwerkettensequenzen, wie jeweils dargestellt in SEQ ID-Nr.: 1, SEQ ID-Nr.: 2 und SEQ ID-Nr.: 3 sowie CDR1-, CDR2- und CDR3-Leichtkettensequenzen, wie jeweils dargestellt in SEQ ID-Nr.: 4, SEQ ID-Nr.: 5 und SEQ ID-Nr.: 6.

2. Antikörper oder Fragment nach Anspruch 1, der bzw. das variable Schwer- und Leichtkettenregionen umfasst, wie jeweils dargestellt in SEQ ID-Nr.: 7 und SEQ ID-Nr.: 8.

3. Antikörper oder Fragment nach Anspruch 1 oder 2, der bzw. das ein konformes Epitop bindet, das durch die drei Proteine UL128, UL130 und UL131A gebildet wird.

4. Antikörper oder Fragment nach einem der Ansprüche 1 - 3, wobei der Antikörper ein monoklonaler Antikörper, ein menschlicher monoklonaler Antikörper, ein Einzelketten-Antikörper, Fab, Fab', F (ab')₂, Fv oder scFv ist.

5. Isoliertes Nukleinsäuremolekül, das eine Nukleotidsequenz umfasst, die den Antikörper oder das Fragment nach einem der vorhergehenden Ansprüche kodiert.

6. Nukleinsäuremolekül nach Anspruch 5, das Folgendes umfasst: die Nukleotidsequenz von einer der folgenden SEQ ID-Nummern: 9, 10, 11, 12, 13, 14, 15 oder 16 oder eine Nukleotidsequenz, die dieselbe Aminosäuresequenz kodiert, wie die Nukleotidsequenz von einer der folgenden SEQ ID-Nummern: 9, 10, 11, 12, 13, 14, 15 oder 16.

7. Expressionsvektor, der ein Nukleinsäuremolekül nach Anspruch 5 oder 6 umfasst.

8. Zelle, die mit dem Vektor nach Anspruch 7 transformiert ist.

9. Pharmazeutische Zusammensetzung, die den Antikörper oder das Fragment nach einem der Ansprüche 1 - 4 und ein pharmazeutisch akzeptables Verdünnungsmittel oder einen pharmazeutisch akzeptablen Träger umfasst.

10. Zusammensetzung nach Anspruch 9, die ferner einen zweiten Antikörper gegen hCMV oder ein Antigen-bindendes Fragment davon umfasst.

11. Zusammensetzung nach Anspruch 9, die ferner Folgendes umfasst:
(a) einen zweiten Antikörper oder ein Antigen-bindendes Fragment davon, der bzw. das sich an ein hCMV-Protein bindet, das aus Folgendem ausgewählt wird: gB, gH, gL, gM, gN, gO, UL128, UL130 und UL131A oder einer Kombination davon; oder
(b) einen zweiten Antikörper oder ein Antigen-bindendes Fragment davon, der bzw. das sich an das hCMV-Protein gB bindet.

12. Verwendung des Antikörpers oder Fragments nach einem der Ansprüche 1 - 4, der Nukleinsäure nach Anspruch 5 oder 6 oder der Zelle nach Anspruch 8 oder der Zusammensetzung nach einem der Ansprüche 9 bis 11 zur Herstellung eines Medikaments zur Prävention oder Behandlung von hCMV-Infektionen.

13. Antikörper oder Fragment nach einem der Ansprüche 1 bis 4 oder Zusammensetzung nach einem der Ansprüche 9 bis 11 zur Verwendung bei der Prävention oder Behandlung von hCMV-Infektionen.

14. Zusammensetzung, die einen Antikörper oder ein Fragment nach einem der Ansprüche 1 bis 4 umfasst, zur Verwendung bei der Prävention oder Behandlung von hCMV-Infektionen, wobei die Zusammensetzung ferner einen zweiten Antikörper umfasst, der aus Folgendem ausgewählt wird:
(a) einem Antikörper oder Antigen-bindenden Fragment davon gegen HCMV;
(b) einem Antikörper oder Antigen-bindenden Fragment davon, der bzw. das an ein hCMV-Protein bindet, das aus Folgendem ausgewählt wird: gB, gH, gL, gM, gN, gO, UL128, UL130 und UL131A oder einer Kombination davon; und
(c) einem Antikörper oder ein Antigen-bindenden Fragment davon, der bzw. das sich an das hCMV-Protein gB bindet.

15. Verfahren zur Herstellung des Antikörpers oder Fragments nach einem der Ansprüche 1 - 4, das (i) das Kultivieren der Zelle nach Anspruch 8 und (ii) das Isolieren des Antikörpers oder Fragments umfasst.

## Revendications

1. Anticorps isolé, ou fragment de liaison à un antigène de celui-ci, qui est spécifique à une combinaison de protéines du hCMV UL128, UL130 et UL131A, la combinaison formant l'épitope reconnu par ledit anticorps ou fragment de liaison, et qui neutralise l'infection de cellules endothéliales, de cellules rétiniennes ou de cellules dendritiques par le cytomégalovirus humain (hCMV), l'anticorps ou fragment comprenant les séquences de chaîne lourdes CDR1, CDR2 et CDR3 telles que définies dans SEQ ID n° : 1, SEQ ID n° : 2 et SEQ ID n° : 3, respectivement, et les séquences de chaîne légère CDR1, CDR2 et CDR3 telles que définies dans SEQ ID N° : 4, SEQ ID n° : 5 et SEQ ID n° : 6, respectivement.

2. Anticorps ou fragment de la revendication 1, comprenant des régions variables de chaînes lourdes et légères telles que définies dans SEQ ID n° : 7 et SEQ ID n° : 8, respectivement.

3. Anticorps ou fragment de la revendication 1 ou 2, qui se line à un épitope conformationnel formé par les trois protéines UL128, UL130 et UL131 A.

4. Anticorps ou fragment de l'une quelconque des revendications 1 à 3, dans lequel l'anticorps est un anticorps monoclonal, un anticorps monoclonal humain, un anticorps monochaîne, Fab, Fab', F(ab')₂, Fv ou scFv.

5. Molécule d'acide nucléique isolé comprenant une séquence nucléotidique codant pour l'anticorps ou fragment de l'une quelconque des revendications précédentes.

6. Molécule d'acide nucléique de la revendication 5 comprenant la séquence nucléotidique de l'une quelconque de SEQ ID n° : 9, 10, 11, 12, 13, 14, 15 ou 16, ou une séquence nucléotidique codant pour la même séquence d'acide aminés que la séquence nucléotidique de l'une quelconque de SEQ ID n° : 9, 10, 11, 12, 13, 14, 15 ou 16.

7. Vecteur d'expression comprenant une molécule d'acide nucléique selon la revendication 5 ou 6.

8. Cellule transformée avec le vecteur selon la revendication 7.

9. Composition pharmaceutique comprenant l'anticorps ou fragment de l'une quelconque des revendications 1 à 4 et diluant ou support pharmaceutiquement acceptable.

10. Composition de la revendication 9, comprenant en outre un second anticorps dirigé contre le hCMV, ou un fragment de liaison à un antigène de celui-ci.

11. Composition de la revendication 9, comprenant en outre
(a) un second anticorps, ou un fragment de liaison à un antigène de celui-ci, qui se lie à une protéine du hCMV choisie parmi gB, gH, gL, gM, gN, gO, UL128, UL130 et UL131A, ou une combinaison de celles-ci ; ou
(b) un second anticorps, ou un fragment de liaison à un antigène de celui-ci, qui se lie à la protéine du hCMV gB.

12. Utilisation de l'anticorps ou fragment de l'une quelconque des revendications 1 à 4, de l'acide nucléique de la revendication 5 ou 6, ou de la cellule de la revendication 8, ou de la composition de l'une quelconque des revendications 9 à 11, dans la fabrication d'un médicament destiné à la prévention ou au traitement d'une infection par hCMV.

13. Anticorps ou fragment de l'une quelconque des revendications 1 à 4 ou composition de l'une quelconque des revendications 9 à 11, à utiliser dans la prévention ou le traitement d'une infection par hCMV.

14. Composition comprenant un anticorps ou fragment de l'une quelconque des revendications 1 à 4 à utiliser dans la prévention ou le traitement d'une infection par hCMV, la composition comprenant en outre un second anticorps choisi parmi :
(a) un anticorps ou un fragment de liaison à un antigène de celui-ci dirigé contre le hCMV ;
(b) un anticorps ou un fragment de liaison à un antigène de celui-ci qui se lie à une protéine du hCMV choisie parmi gB, gH, gL, gM, gN, gO, UL128, UL130 et UL131A, ou une combinaison de celles-ci ; et
(c) un anticorps ou un fragment de liaison à un antigène de celui-ci qui se lie à la protéine du hCMV gB.

15. Procédé de production de l'anticorps ou fragment de l'une quelconque des revendications 1 à 4, comprenant (i) la mise en culture de la cellule de la revendication 8 et (ii) l'isolement de l'anticorps ou fragment.


### Sequence listing

### SEQUENCE LISTING

<110> HUMABS LLC
<120> HUMAN CYTOMEGALOVIRUS NEUTRALISING ANTIBODIES AND USE THEREOF
<130> P051016WO
<160> 16
<170> SeqWin99, version 1.02
<210> 1
   <211> 7
   <212> PRT
   <213> Homo sapiens
<400> 1
   Gly Tyr Arg Thr Ser Tyr Tyr
   1               5
<210> 2
   <211> 5
   <212> PRT
   <213> Homo sapiens
<400> 2
   Tyr Gly Asp Ser Asp
   1               5
<210> 3
   <211> 12
   <212> PRT
   <213> Homo sapiens
<400> 3
   Ala Arg Ser Thr Ser Gly Asp Tyr Val Gly Ala Asp
   1               5                   10
<210> 4
   <211> 7
   <212> PRT
   <213> Homo sapiens
<400> 4
   Ser Val Tyr Ser Asp Asp Asn
   1               5
<210> 5
   <211> 3
   <212> PRT
   <213> Homo sapiens
<400> 5
   Lys Val Ser
   1
<210> 6
   <211> 6
   <212> PRT
   <213> Homo sapiens
<400> 6
   Met Gly Arg His Trp Thr
   1               5
<210> 7
   <211> 93
   <212> PRT
   <213> Homo sapiens
<400> 7
   Val Val Ser Gly Ala Val Lys Lys Gly Lys Ser Arg Ser Cys Lys Ala
   1               5                   10                  15
   Ser Gly Tyr Arg Thr Ser Tyr Tyr Ala Trp Val Arg His Met Gly Lys
               20                  25                  30
   Gly Trp Met Gly Tyr Gly Asp Ser Asp Thr Tyr Ser Ser Gly Val Thr
           35                  40                  45
   Ser Ala Asp Lys Ser Ala Thr Thr Ala Tyr Trp Ser Ser Arg Ala Ser
       50                  55                  60
   Asp Thr Ala Met Tyr Tyr Cys Ala Arg Ser Thr Ser Gly Asp Tyr Val
   65                  70                  75                  80
   Gly Ala Asp Trp Gly Gly Thr Met Val Thr Val Ser Ser
                   85                  90
<210> 8
   <211> 75
   <212> PRT
   <213> Homo sapiens
<400> 8
   Asp Val Thr Ser Ser Ala Val Thr Gly Ala Ser Ser Cys Arg Ser Asn
   1               5                   10                  15
   Ser Val Tyr Ser Asp Asp Asn Asn Trp Gly Gly Arg Arg Tyr Lys Val
               20                  25                  30
   Ser Asn Arg Asp Ser Gly Val Asp Arg Ser Gly Ser Gly Ser Gly Thr
           35                  40                  45
   Asp Thr Lys Ser Arg Val Ala Asp Val Gly Val Tyr Tyr Cys Met Gly
       50                  55                  60
   Arg His Trp Thr Gly Gly Thr Lys Val Asp Lys
   65                  70                  75
<210> 9
   <211> 373
   <212> DNA
   <213> Homo sapiens
<400> 9
   gaggtgcagc tggtgcagtc tggagcagag gtgaaaaagc ccgggaagtc tctgaggatc 60
   tcctgtaagg cttctggata caggtttacc agctactaca tcgcctgggt gcgccacatg 120
   cccgggaaag gcctggagtg gatggggatc atctatcctg gtgactctga tatcacatac 180
   agcccgtcct tccaaggcca ggtcaccatc tcagccgaca agtccgccac taccgcctac 240
   ctgcaatgga gcagcctgag ggcctcggac accgccatgt actactgtgc gagactctca 300
   ttaacagagt ccggtgacta cgtcggtgcg tttgatatct ggggccaagg gacaatggtc 360
   accgtctctt cag 373
<210> 10
   <211> 343
   <212> DNA
   <213> Homo sapiens
<400> 10 cctctattca ctttcggccc tgggaccaaa gtggatatca aac 343
<210> 11
   <211> 24
   <212> DNA
   <213> Homo sapiens
<400> 11
   ggatacaggt ttaccagcta ctac 24
<210> 12
   <211> 24
   <212> DNA
   <213> Homo sapiens
<400> 12
   atctatcctg gtgactctga tatc 24
<210> 13
   <211> 51
   <212> DNA
   <213> Homo sapiens
<400> 13
   gcgagactct cattaacaga gtccggtgac tacgtcggtg cgtttgatat c 51
<210> 14
   <211> 33
   <212> DNA
   <213> Homo sapiens
<400> 14
   caaagcctcg tatacagtga tgacaacatc ttc 33
<210> 15
   <211> 9
   <212> DNA
   <213> Homo sapiens
<400> 15
   aaggtttct 9
<210> 16
   <211> 33
   <212> DNA
   <213> Homo sapiens
<400> 16
   atgcaaggta gacactggcc tcctctattc act 33